# EUROPÄISCHE PATENTSCHRIFT

(11) **EP 1 517 891 B1**
(45) Veröffentlichungstag und Bekanntmachung des Hinweises auf die Patenterteilung: **03.04.2013**
(21) Anmeldenummer: 03735535.1
(22) Anmeldetag: 04.06.2003
(51) Int. Cl.: C07D 205/08, C07F 9/568, A61K 31/397, A61P 3/06, A61P 9/10

(54) **SÄUREGRUPPEN SUBSTITUIERTE DIPHENYLAZETIDINONE, VERFAHREN ZU DEREN HERSTELLUNG, DIESE VERBINDUNGEN ENTHALTENDE ARZNEIMITTEL UND DEREN VERWENDUNG**
DIPHENYL AZETIDINONES SUBSTITUTED BY ACIDIC GROUPS, METHOD FOR THEIR PRODUCTION, MEDICAMENTS CONTAINING SAID COMPOUNDS AND USE THEREOF
DIPHENYLAZETINIDONES SUBSTITUEES PAR DES GROUPES ACIDES, PROCEDE DE FABRICATION, MEDICAMENTS CONTENANT CES COMPOSES ET UTILISATION DE CES MEDICAMENTS

(30) Priorität: 19.06.2002 DE 10227508
(43) Veröffentlichungstag der Anmeldung: 30.03.2005
(73) Patentinhaber: Sanofi-Aventis Deutschland GmbH, 65929 Frankfurt am Main (DE)
(72) Erfinder: JAEHNE, Gerhard, 65929 Frankfurt (DE); FRICK, Wendelin, 65510 Hünstetten-Beuerbach (DE); FLOHR, Stefanie, CH-4054 Basel (CH); LINDENSCHMIDT, Andreas, 65812 Bad Soden (DE); GLOMBIK, Heiner, 65719 Hofheim (DE); KRAMER, Werner, 55130 Mainz-Laubenheim (DE); HEUER, Hubert, 55270 Schwabenheim (DE); SCHAEFER, Hans-Ludwig, 65239 Hochheim (DE)
(86) Internationale Anmeldenummer: PCT/EP2003/005816
(87) Internationale Veröffentlichungsnummer: WO 2004/000805

(56) Entgegenhaltungen:
- WO-A-02/18432
- WO-A-02/50027
- WO-A-02/50060
- WO-A-02/50068
- US-A- 5 767 115

## Beschreibung

Die Erfindung betrifft mit Säuregruppen substituierte Diphenylazetidinone, deren physiologisch verträgliche Salze sowie physiologisch funktionelle Derivate.

Es sind bereits Diphenylazetidinone (wie z.B. Ezetimibe) sowie deren Verwendung zur Behandlung von Hyperlipidämie sowie Arteriosklerose und Hypercholesterinämie beschrieben worden [vgl. Drugs of the Future 2000, 25(7):679-685) und US 5,756,470].

Der Erfindung lag die Aufgabe zugrunde, weitere Verbindungen zur Verfügung zu stellen, die eine therapeutisch verwertbare hypolipidämische Wirkung entfalten. Insbesondere bestand die Aufgabe darin, neue Verbindungen zu finden, die gegenüber den im Stand der Technik beschriebenen Verbindungen, sehr gering resorbierbar sind. Unter sehr gering resorbierbar wird eine intestinale Resorption kleiner 10%, bevorzugt kleiner oder gleich 5% verstanden.

Die neuen Verbindungen sollen insbesonders eine geringere Resorption als Ezetimibe auf weisen.
Bei geringerer Resorption zeigen pharmazeutische Wirkstoffe in der Regel deutlich weniger Nebenwirkungen.

Die Erfindung betrifft daher Verbindungen der Formel I, 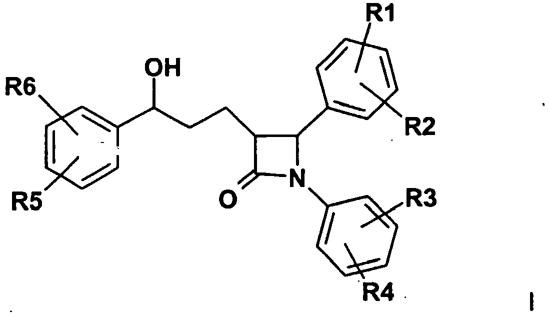 worin bedeuten
- R1, R2, R3, R4, R5, R6: unabhängig voneinander (C₀-C₃₀)-Alkylen-(LAG)ₙ, wobei n = 1 - 5 sein kann und wobei ein oder mehrere C-Atome des Alkylenrests durch -S(O)ₙ-, mit n = 0 - 2, -O-, -(C=O)-, -(C=S)-, -CH=CH-, -CΞC-, -N((C₁-C₆)-Alkyl)-, -N(Phenyl)-, -N((C₁-C₆)-Alkyl-Phenyl)-, -N(CO-(CH₂)₁₋₁₀-COOH)- oder -NH- ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- (LAG)ₙ: -(CH₂)₁₋₁₀-SO₃H, -(CH₂)₀₋₁₀-P(O)(OH)₂, (CH₂)₀₋₁₀-O-P(O)(OH)₂, -(CH₂)₀₋₁₀-COOH und n = 1 - 5 sein kann;
wobei immer mindestens einer der Reste R1 bis R6 die Bedeutung (C₀-C₃₀) Alkylen-(LAG)ₙ, wobei n = 1 - 5 ist und wobei ein oder mehrere C-Atome des Alkylenrests durch -S(O)ₙ-, mit n = 0 - 2, -O-, -(C=O)-, -(C=S)-, -CH=CH-, -CΞC-, - N((C₁-C₆)-Alkyl)-, -N(Phenyl)-, -N((C₁-C₆)-Alkyl-Phenyl)-, -N(CO-(CH₂)₁₋₁₀-COOH)- oder -NH- ersetzt sein können,
besitzen muß,
sowie deren pharmazeutisch verträglichen Salze;
wobei die Verbindung 2-{[4-(4-{1-(4-Fluor-phenyl)-3-[3-(4-fluor-phenyl)-3-hydroxypropyl]-4-oxo-azetidin-2-yl}-phenoxy)-butyl]-methyl-amino}-ethansulfonsäure sowie solche Verbindungen, bei welchen die Reste R1 - R6 die Bedeutung -O-(CH2)₁₋₁₀-COOH, (C₁-C₆)-Alkylen-COOH oder -COOH haben, ausgenommen sind.

Bevorzugt sind Verbindungen der Formel I, worin mindestens einer der Reste R1 bis R6 die Bedeutung (C₀-C₃₀)-Alkylen-(LAG), wobei ein oder mehrere C-Atome des Alkylenrests durch -O-, -(C=O)-, -N((C₁-C₆)-Alkyl)-, -N(CO-(CH₂)₁₋₁₀-COOH)- oder - NH- ersetzt sein können, besitzt.

Besonders bevorzugt sind Verbindungen der Formel I, worin einer der Reste R1 oder R3 die Bedeutung (C₀-C₃₀)-Alkylen-(LAG) hat, wobei ein oder mehrere C-Atome des Alkylenrests durch -O-, -(C=O)-, -N(CH₃)-, oder -NH- ersetzt sein können.

Ganz besonders bevorzugt sind Verbindungen der Formel I, worin einer der Reste R1 oder R3 die Bedeutung -(CH₂)₀₋₁-Y-W-(C₀-C₂₅)-Alkylen-Y'-W'-(LAG) hat; worin ein oder mehrere C-Atome des Alkylenrests durch O-Atome ersetzt sein können und wobei Y und W unabhängig voneinander NH, NCH₃, C=O, O, eine Bindung oder S(O)ₙ, mit n = 0 - 2, sein können und Y' und W' unabhängig voneinander NH, NCH₃, C=O, O, eine Bindung oder S(O)ₙ, mit n = 0 - 2, sein können oder Y-W oder Y'-W' jeweils für sich zusammen genommen eine Bindung sein kann.

Weiterhin bevorzugt sind Verbindungen der Formel I, worin die Gruppe LAG ein Carbonsäurerest oder ein Sulfonsäurerest ist.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bemstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Verbindungen der Formel I und deren pharmazeutisch verträgliche Salze stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.

Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Diphenylazetidinon-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Antidiabetika umfassen Insulin und Insulinderivate, wie z.B. Lantus^{®} oder HMR 1964, GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlididämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor , wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramin, Colesolvam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.
Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylhamstoff, wie z.B. Tolbutamid, Glibenclamid, Glipizid oder Gliclazid, verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinyl-methoxy]phenyl]methyl]-2,4-thiazolidindion, verabreicht.
Bei einer Ausführungsform werden die Verbindungen der Formel I in Kombination mit einem *α*-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Gliazid oder Repaglinid.
Bei einer Ausführungsform werden die Verbindungen der Formel I in Kombination mit mehr als einer der vorstehend genannten Verbindungen, z.B. in Kombination mit einem Sulphonylhamstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Agonisten, NPY-Agonisten, MC-3- oder MC-4-Agonisten, Orexin-Agonisten, H3-Agonisten, TNF-Agonisten, CRF-Agonisten, CRF BP-Antagonisten, Urocortin-Agonisten, *β*3-Agonisten, MCH (Melanin-konzentrierendes Hormon) Antagonisten, , CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-*β-*Agonisten verabreicht.

Bei einer Ausführungsform der Erfindung ist der weitere Wirkstoff Leptin.
Bei einer Ausführungsform ist der weitere Wirkstoff Dexamphatamin oder Amphetamin.
Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen, wie z.B. Caromax^{®} verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden. Die Kombination von Verbindungen der Formel I mit Caromax^{®} zeichnet sich neben einer Wirkverbesserung, insbesonders in der LDL-Cholesterinsenkung, gegenüber den Einzelwirkstoffen, auch durch Ihre verbesserte Verträglichkeit aus.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

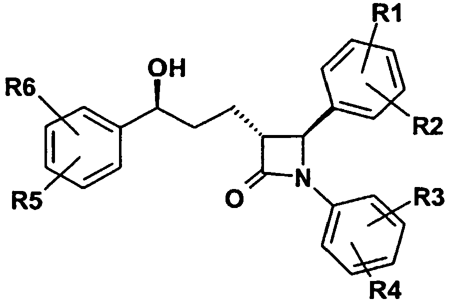

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl- (Trt)-Rest, der durch Säuren wie 3M Salzsäure abspaltbare t-Butylcarbamat (BOC-)-Rest und der durch sekundäre Amine abspaltbare 9-Fluorenylmethyloxycarbonyl- (Fmoc)-Rest herangezogen.

Die Erfindung betrifft weiterhin ein Verfahren zur Herstellung von Diphenylazetidinonderivaten der Formel I. 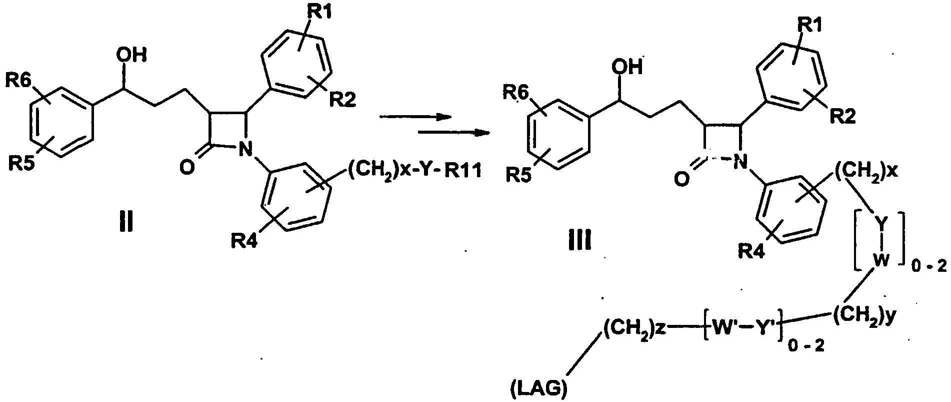 Y kann S, O, (C=O), (C=S), CH=CH, CΞC, N((C₁-C₆)-Alkyl), N(Phenyl), N((C₁-C₆)-Alkyl-Phenyl), N(CO-(CH₂)₁₋₁₀-COOH) oder NH bedeuten;
R11 kann H oder im Falle, dass Y = (C=O) oder (C=S) ist, OH bedeuten; W, Y' und W' können, unabhängig voneinander und von Y, -S(O)ₙ-, mit n = 0 - 2, -O-, -(C=O)-, -(C=S)-, -CH=CH-, -CΞC-, -N((C₁-C₆)-Alkyl)-, -N(Phenyl), -N((C₁-C₆)AlkylPhenyl)-, -N(CO-(CH2)₁₋₁₀-COOH)- oder -NH- oder eine Bindung bedeuten;
x, y und z können unabhängig voneinander 0 bis 10 bedeuten.

Die Verknüpfung von -(CH₂)x-Y-R11 in Verbindung II kann alternativ auch an einem der anderen beiden Phenylringen sein.

Das Verfahren zur Herstellung der Verbindungen der Formel I ist dadurch gekennzeichnet, daß man z.B. ein Amin oder eine Hydroxy-Verbindung der Formel II mit einem Alkylierungs- oder einem Acylierungsreagenz umsetzt, das bevorzugt in omega-Position eine weitere Funktionalität - evtl. in geschützter Form - trägt. Diese wird (nach Entschützung) zur Anknüpfung der (LAG) verwendet, beispielsweise unter Ausbildung von Ether-, Amin oder Amidbindungen.

Die nachfolgenden Beispiele dienen zur näheren Erläuterung der Erfindung, ohne dieselbe auf in den Beispielen beschriebene Produkte und Ausführungsformen einzuschränken.

### Beispiel I

### 4-{4-[3-[3-(4-Fluor-phenyl)-3-hydroxy-propyl]-2-(4-methoxy-phenyl)-4-oxo-azetidin-1-yl]-benzylamino}-butane-1-sulfonsäure (6)

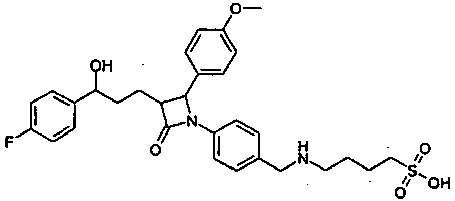

### a) 3-[5-(tert-Butyl-dimethyl-silanyloxy)-5-(4-fluor-phenyl)-pentanoyl]-4-phenyl-oxazolidin-2-on (1)

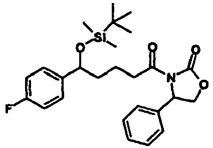

27 g 3-[5-(4-Fluor-phenyl)-5-hydroxy-pentanoyl]-4-phenyl-oxazolidin-2-on werden mit 13,6 g Tert.-Butyl-Dimethylsilylchlorid und 10,2 g Imidazol in 36 ml Dimethylformamid gelöst und 90 min. bei 60°C gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Man erhält 3-[5-(tert-Butyl-dimethyl-silanyloxy)-5-(4-fluor-phenyl)-pentanoyl]-4-phenyl- oxazolidin-2-on (1) mit dem Molekulargewicht 471,65 (C₂₆H₃₄FNO₄Si); MS (ESI): 340.28 (MH⁺- HOSi(CH₃)₂C(CH₃)₃).

### b) 4-[5-(tert-Butyl-dimethyl-silanyloxy)-5-(4-fluor-phenyl)-1-(4-methoxy-phenyl)-2-(2-oxo-4-phenyl-oxazolidin-3-carbonyl)-pentylamino]-benzonitril (2)

16,2 g 3-[5-(tert-Butyl-dimethyl-silanyloxy)-5-(4-fluor-phenyl)-pentanoyl]-4-phenyl-oxazolidin-2-on werden in 350 ml Dichlormethan gelöst. Die Lösung wird mit 19,8 ml Hünig Base und mit 10,14 g 4-[(4-Methoxy-phenylimino)-methyl]-benzonitril versetzt und auf -10°C gekühlt. Zur gekühlten Lösung fügt man 8,52 ml. Trimethylsilyltriflat hinzu und rührt 30 min. bei -10°C. Die Lösung wird nun auf -30°C abgekühlt, und es werden 44 ml Titantetrachloridlösung zugegeben. Die Reaktionsmischung wird 2 h bei -30 bis-40°C gerührt. Danach lässt man die Lösung sich auf Raumtemperatur erwärmen, wäscht die Reaktionslösung nacheinander mit 200 ml 2N Schwefelsäure, 300 ml 20%iger Natriumhydrogensulfitllösung und ges. Kochsalzlösung. Die organische Phase wird über Magnesiumsulfat getrocknet, im Vakuum eingeengt und der Rückstand wird über Kieselgel mit n-Heptan/Essigsäureethylester 3/1 gereinigt. Man erhält 4-[5-(tert-Butyl-dimethyl-silanyloxy)-5-(4-fluor-phenyl)-1-(4-methoxyphenyl)-2-(2-oxo-4-phenyl-oxazolidin-3-carbonyl)-pentylamino]-benzonitril (2) mit dem Molekulargewicht 707,93 (C₄₁H₄₆FN₃O₅Si); MS (ESI): 590.51 (MH⁺- C₇H₅N₂).

### c) 4-[3-[3-(tert-Butyl-dimethyl-silanyloxy)-3-(4-fluor-phenyl)-propyl]-2-(4-methoxyphenyl)-4-oxo-azetidin-1-yl]-benzonitril (3)

13,2 g 4-[5-(tert-Butyl-dimethyl-silanyloxy)-5-(4-fluor-phenyl)-1-(4-methoxy-phenyl)-2-(2-oxo-4-phenyl-oxazolidin-3-carbonyl)-pentylamino]-benzonitril werden in 380 ml Methyl-tert.-Butylether gelöst, mit 18,6 ml N,O-Bis(trimethylsilyl)-acetamid und 1,86 ml einer 1 M Lösung von Tetrabutylammoniumfluorid in Tetrahydrofuran versetzt und 2 h bei Raumtemperatur gerührt. Nach Beendigung der Reaktion fügt man 10 ml Essigsäure zu, engt die Reaktionsmischung im Vakuum ein und reinigt den Rückstand über Kieselgel mit Toluol/Essigsäureethylester 50/1. Man erhält 4-[3-[3-(tert-Butyl-dimethyl-silanyloxy)-3-(4-fluor-phenyl)-propyl]-2-(4-methoxy-phenyl)-4-oxo-azetidin-1-yl]-benzonitril (3) mit dem Molekulargewicht 544,75 (C₃₂H₃₇FN₂O₃Si); MS (ESI): 545.56 (M+H⁺).

### d) 4-[3-[3-(4-Fluor-phenyl)-3-hydroxy-propyl]-2-(4-methoxy-phenyl)-4-oxo-azetidin-1-yl]- benzonitril (4)

3.5 g 4-[3-[3-(tert-Butyl-dimethyl-silanyloxy)-3-(4-fluor-phenyl)-propyl]-2-(4-methoxyphenyl)-4-oxo-azetidin-1-yl]-benzonitril werden in 65 ml Tetrahydrofuran gelöst, mit 0,74 ml Essigsäure und 8,03 ml einer 1 M Lösung von Tetrabutylammoniumfluorid in Tetrahydrofuran versetzt und 2 h bei Raumtemperatur gerührt. Danach werden 4,82 ml der Tetrabutylammoniumfluorid-Lösung nachgegeben und weitere 3 h bei Rückflusstemperatur gerührt. Die abgekühlte Reaktionsmischung wird im Vakuum eingeengt, und der Rückstand wird chromatographisch über Kieselgel mit n-Heptan/Essigsäureethylester 2/1 gereinigt. Man erhält 4-[3-[3-(4-Fluor-phenyl)-3-hydroxy-propyl]-2-(4-methoxy-phenyl)-4-oxo-azetidin-1-yl]- benzonitril (4) mit dem Molekulargewicht 430,48 (C₂₆H₂₃FN₂O₃); MS (ESI): 431.24 (M+H⁺).

### e) 1-(4-Aminomethyl-phenyl)-3-[3-(4-fluor-phenyl)-3-hydroxy-propyl]-4-(4-methoxyphenyl)- azetidin-2-on (5)

1,22 g 4-[3-[3-(4-Fluor-phenyl)-3-hydroxy-propyl]-2-(4-methoxy-phenyl)-4-oxo-azetidin-1-yl]- benzonitril werden in 90 ml Ethanol gelöst, mit 10 ml konz. Ammoniaklösung und einem Überschuß Raney-Nickel versetzt und 8 h bei 60°C und einem Druck von 10 bar Wasserstoff gerührt. Die Reaktionsmischung kühlt über Nacht auf Raumtemperatur ab; anderntags wird vom Katalysator abgetrennt, das Filtrat im Vakuum eingeengt und der Rückstand chromatographisch über Kieselgel mit Dichlormethan/Methanol/Ammoniak-Lösung 10/1/0.1 gereinigt. Man erhält 1-(4-Aminomethyl-phenyl)-3-[3-(4-fluor-phenyl)-3-hydroxy-propyl]-4-(4-methoxy-phenyl)-azetidin-2-on (5) mit dem Molekulargewicht 434,51 (C₂₆H₂₇FN₂O₃); MS (ESI): 418.2 (MH⁺- NH₃).

### f) 4-{4-[3-[3-(4-Fluor-phenyl)-3-hydroxy-propyl]-2-(4-methoxy-phenyl)-4-oxo-azetidin-1-yl]-benzylamino}-butane-1-sulfonsäure (6)

87 mg des obigen Benzylamins werden bei Raumtemperatur in 3 ml trockenem Acetonitril gelöst, mit 40 µl 1,4-Butansulton versetzt und 12 h unter Rückfluss erhitzt. Die abgekühlte Reaktionslösung wird im Vakuum eingeengt und chromatographisch (Kieselgel; Dichlormethan/Methanol 85/15 + 10% Wasser) gereinigt. Man erhält 4-{4-[3-[3-(4-Fluor-phenyl)-3-hydroxy-propyl]-2-(4-methoxy-phenyl)-4-oxo-azetidin-1-yl]-benzylamino}-butane-1-sulfonsäure (6) mit dem Molekulargewicht 570,69 (C₃₀H₃₅FN₂O₆S); MS (ESI): 553,28 (MH⁺- H₂O).

### Beispiel II

### 2-[(4-{4-[3-[3-(4-Fluor-phenyl)-3-hydroxy-propyl]-2-(4-methoxy-phenyl)-4-oxo-azetidin-1- yl]-phenoxy}-butyl)-methyl-amino]-ethylsulfonsäure (8):

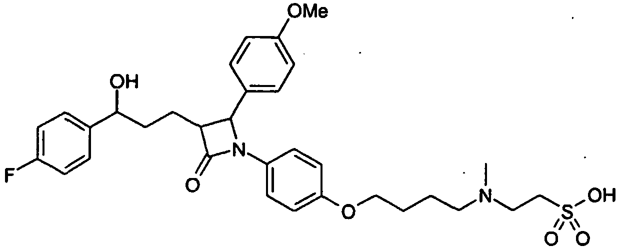

In 6 ml absolutem Methanol werden 130 mg 3-[3-(4-Fluor-phenyl)-3-hydroxy-propyl]-1-[4-(4-fluor-butoxy)-phenyl]-4-(4-methoxy-phenyl)-azetidin-2-on (7) gelöst. Dann werden 120 mg N-Methyltaurin in 2 ml Wasser und 60 mg Kaliumcarbonat zugegeben. Bei 50 °C wird 24 h lang gerührt. Die Reaktionsmischung wird am Rotationsverdampfer eingeengt und der Rückstand über präparative Chromatographie gereinigt. Nach Gefriertrocknung wird das Produkt (50 mg) als Öl erhalten.
C₃₂H₃₉FN₂O₇S ESIMS m/z: 614 (M⁺)

### Beispiel III

### [2-(4-{4-[3-[3-(4-Fluor-phenyl)-3-hydroxy-propyl]-2-(4-methoxy-phenyl)-4-oxo-azetidin-1- yl]-phenoxy}-butylamino)-ethyl]-phosphonsäure (9):

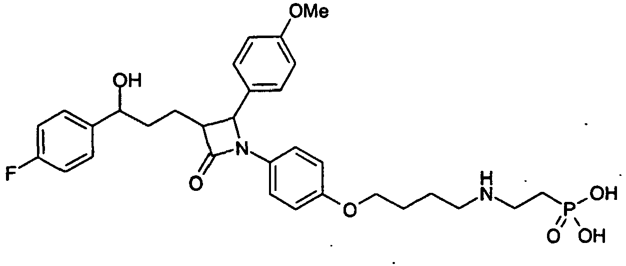

In 6 ml absolutem Methanol werden 200 mg 3-[3-(4-Fluor-phenyl)-3-hydroxy-propyl]-1-[4-(4-fluor-butoxy)-phenyl]-4-(4-methoxy-phenyl)-azetidin-2-on (7) gelöst. Dann werden 165 mg 1-Aminoethylphosphat und 247 mg Kaliumcarbonat in 3 ml Wasser gelöst zugegeben. Bei 90 °C wird 8 h lang gerührt. Die Reaktionsmischung wird am Rotationsverdampfer eingeengt und der Rückstand über präparative Chromatographie gereinigt. Nach Gefriertrocknung wird das Produkt (47 mg) als Öl erhalten.
C₃₁H₃₈FN₂O₇P ESIMS m/z: 600 (M⁺)

### Beispiel IV

### Phosphorsäure-mono-{6-[4-(4-{1-(4-fluor-phenyl)-3-[3-(4-fluorfluor-phenyl)-3-hydroxypropyl]-4-oxo-azetidin-2-yl}-phenoxy)-butylamino]-hexyl} ester (10):

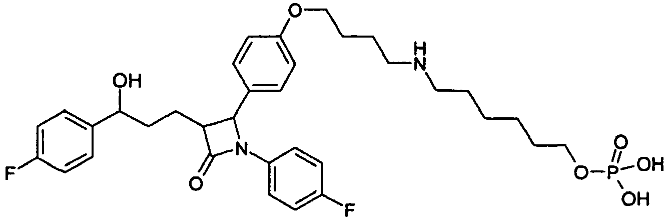

In 6 ml absolutem Methanol werden 115 mg 1-(4-FluorFluor-phenyl)-3-[3-(4-fluorfluor-phenyl)-3-hydroxy-propyl]-4-[4-(2-fluoromethoxy-ethoxy)-phenyl]-azetidin-2-on (7) gelöst. Dann werden 130 mg 6-Amino-1-hexylphosphat in 1,5 ml Wasser und 107 mg Kaliumcarbonat zugegeben. Bei 70 °C wird über Nacht gerührt. Die Reaktionsmischung wird am Rotationsverdampfer eingeengt und der Rückstand über präperative Chromatographie gereinigt. Nach Gefriertrocknung wird das Produkt als Öl erhalten.
C₃₄H₄₃F₂N₂O₇P ESIMS m/z: 660 (M⁺)

### Beispiel V

### 4-{4-[3-[3-(4-FluorFluor-phenyl)-3-hydroxy-propyl]-2-(4-methoxy-phenyl)-4-oxo-azetidin-1-yl]- phenoxy}-butan-1-sulfonsäure (12):

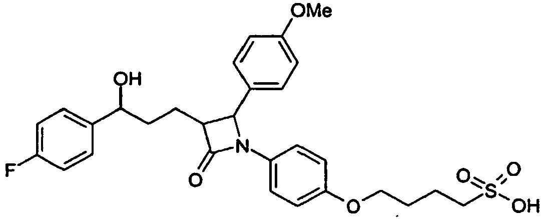

In 4 ml absolutem Dimethylformamid werden 160 mg 3-[3-(4-FluorFluor-phenyl)-3-hydroxy-propyl]-1-(4-hydroxy-phenyl)-4-(4-methoxy-phenyl)-azetidin-2-on (11) gelöst. Es werden 210 mg gepulvertes Kaliumcarbonat und 42 mg 1,4,-Butansulton zugegeben. Bei Raumtemperatur wird über Nacht gerührt. Die Reaktionslösung wird unter Ölpumpenvakuum eingeengt, mit Dichlormethan aufgenommen und 1 x mit Wasser gewaschen. Mit 2N Salzsäure wird die wässrige Phase angesäuert und 2x mit Dichlormethan extrahiert. Die vereinigten organischen Phasen werden über Natriumsulfat getrocknet und eingeengt. Der Rückstand wird über eine 10 g SiO₂-Kartusche chromatographiert (Dichlormethan / Methanol = 5/1). Das Produkt (72 mg) wird als Öl erhalten.
C₂₉H₃₂FNO₇S ESIMS m/z: 557 (M⁺)

### Beispiel VI

### 4-(4-{1-(4-Fluor-phenyl)-3-[3-(4-fluor-phenyl)-3-hydroxy-propyl]-4-oxo-azetidin-2-yl)-phenoxy)-butan-1-sulfonsäure (13):

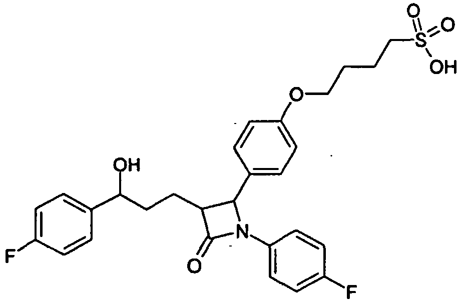

In 6 ml absolutem Dimethylformamid werden 250 mg 1-(4-Fluor-phenyl)-3-[3-(4-fluorphenyl)-3-hydroxy-propyl]-4-(4-hydroxy-phenyl)-azetidin-2-on (7) gelöst. Es werden 337 mg gepulvertes Kaliumcarbonat und 69 µl 1,4,-Butansulton zugegeben. Bei Raumtemperatur wird über Nacht gerührt. Die Reaktionslösung wird filtriert und unter Ölpumpenvakuum eingeengt. Der Rückstand wird über eine 10 g SiO₂-Kartusche chromatographiert (Dichlormethan / Methanol = 5/1) und aus Diethylether kristallisiert. Das Produkt (131 mg) wird als Feststoff erhalten.
C₂₈H₂₉F₂NO₆S ESIMS m/z: 546 (M⁺)

### Beispiel VII

### 3-(4-{1-(4-Fluor-phenyl)-3-[3-(4-fluor-phenyl)-3-hydroxy-propyl]-4-oxo-azetidin-2-yl}-phenoxy)-propan-1-sulfonsäure (14):

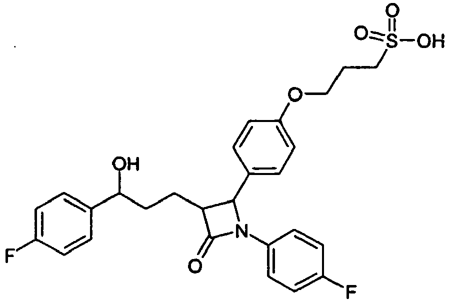

In 6 ml absolutem Dimethylformamid werden 250 mg 1-(4-Fluor-phenyl)-3-[3-(4-fluorphenyl)-3-hydroxy-propyl]-4-(4-hydroxy-phenyl)-azetidin-2-on (7) gelöst. Es werden 337 mg gepulvertes Kaliumcarbonat und 59 µl 1,3,-Propansulton zugegeben. Bei Raumtemperatur wird über Nacht gerührt. Die Reaktionslösung wird filtriert und unter Ölpumpenvakuum eingeengt. Der Rückstand wird über eine 10 g SiO₂-Kartusche chromatographiert (Dichlormethan / Methanol = 5/1) und aus Diethylether kristallisiert. Das Produkt (250 mg) wird als Feststoff erhalten.
C₂₇H₂₇F₂NO₆S ESIMS m/z: 532 (M⁺)

### Beispiel VIII

### (4-{1-(4-Fluor-phenyl)-3-[3-(4-fluor-phenyl)-3-hydroxy-propyl]-4-oxo-azetidin-2-yl}-benzylcarbamoyl)-methansulfonsäure (18):

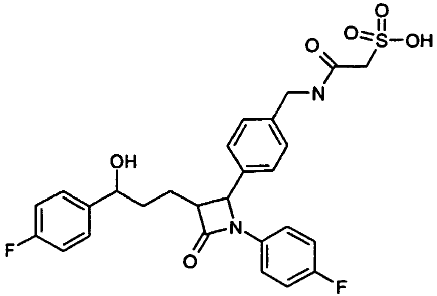

### a) 4-[5-(4-Fluor-phenyl)-1-(4-fluor-phenylamino)-5-hydroxy-2-(2-oxo-4-phenyl-oxazolidin-3- carbonyl)-pentyl]-benzonitril (15):

2.5 g 3-[5-(4-Fluor-phenyl)-5-hydroxy-pentanoyl]-4-phenyl-oxazolidin-2-on werden in 30 ml Dichlormethan unter Argon gelöst, dazu gibt man 3.9 g 4-[(4-Fluor-phenylimino)-methyl]-benzonitril und kühlt auf -10°C.Zu dieser Mischung gibt man 6.4 ml Diisopropylethylamin und innerhalb von 30 min 4.05 ml Trimethylsilylchlorid, so dass die Temperatur -5°C nicht übersteigt. Bei dieser Temp. wird 1 Std. nachgerührt und dann auf -25°C gekühlt. Dann werden 0.8 ml Titantetrachlorid langsam zugegeben. Die dunkle Mischung wird über Nacht bei - 25 bis -30°C gerührt danach mit 35 ml 7proz. Weinsäurelösung zersetzt und 1 Std. bei Raumtemp. nachgerührt. Anschließend gibt man 15 ml einer 20%igen Natriumhydrogencarbonatlösung dazu und rührt erneut 1 Std. Nach Phasentrennung wird die org. Phase mit 30 ml Waser gewaschen, über Magnesiumsulfat getrocknet und auf ca. 10 ml eingeengt. Nach Zugabe von 2 ml Bistrimethylsilylacetamid erwärmt man 30 min. zum Rückfluss und engt danach i.Vak. ein. Der Rückstand wir d mit Ethylacetat/Heptan zur Kristallisation gebracht. Man saugt ab und trocknet i.Vak. Man erhält das Produkt mit dem Molekulargewicht 653.81 (C₃₇H₃₇F₂N₃O₄Si); MS (ESI+): 654.3 (M+H⁺), 582.2 (M+H⁺-Si(CH₃)₃).

### b) {1-(4-Fluor-phenyl)-3-[3-(4-fluor-phenyl)-3-hydroxy-propyl]-4-oxo-azetidin-2-yl}-benzonitril (16):

2 g 4-[5-(4-Fluor-phenyl)-1-(4-fluor-phenylamino)-5-hydroxy-2-(2-oxo-4-phenyl-oxazolidin-3- carbonyl)-pentyl]-benzonitril (15) werden in 20 ml Methyl-tert.-butyl-ether gelöst und mit 100 mg Tetrabutyl- ammoniumfluorid-Trihydrat und 1.3 ml Bistrimethylsilylacetamid ca. 1 h auf 40°C erwärmt. Man verfolgt die Reaktion im Dünnschichtchromatogramm. Nach beendeter Umsetzung setz man zunächst 0.2 ml Eisessig zu , rührt 30 min und engt ein. Der Rückstandwird mit 20 ml einer Mischung von Isopropanol / 2N Schwefelsäure = 10:1 versetzt und 1 Std. gerührt. Nach Zugabe einer Spatelspitze festem Natriumhydrogencarbonat engt man erneut i. Vak. ein, nimmt mit Ethylacetat auf, wäscht die org. Phase mit Wasser , trocknet und reinigt nach Entfernen des Lösemittels den Rückstand durch Säulenchromatographie (SiO₂, CH₂Cl₂/Methanol = 100: 1). Man erhält das Produkt mit dem Molekulargewicht 418.45 (C₂₅H₂₀F₂N₂O₂); MS (DCI+): 419 (M+H⁺).

### c) 4-(4-Aminomethyl-phenyl)-1-(4-fluor-phenyl)-3-[3-(4-fluor-phenyl)-3-hydroxypropyl]- azetidin-2-on (17):

200 mg {1-(4-Fluor-phenyl)-3-[3-(4-fluor-phenyl)-3-hydroxy-propyl]-4-oxo-azetidin-2-yl}-benzonitril (16) werden in 20 ml Ethanol gelöst und mit 0.5 ml konz. Ammoniak über Raney-Nickel 30 Std bei 75 bar Wasserstoff und 25°C hydriert. Man saugt vom Katalysator ab, engt i. Vak. ein und reinigt den Rückstand durch Säulenfiltration (SiO₂, CH₂Cl₂/Methanol/. NH₃ conc = 100:10:1). Man erhält das Produkt mit dem Molekulargewicht 422.5 (C₂₅H₂₂F₂N₂O₂); MS (DCI+): 423 (M+H⁺), 405 (M+H⁺- H₂O).

### d) (4-{1-(4-Fluor-phenyl)-3-[3-(4-fluor-phenyl)-3-hydroxy-propyl]-4-oxo-azetidin-2-yl}-benzylcarbamoyl)-methansulfonsäure (18):

Zu einer Lösung aus 40 mg Sulfoessigsäure, 110 µl Diisopropylcarbodiimid, 76 mg Hydroxybenzotriazol in 2 ml Dimethylformamid wird eine Lösung aus 120 mg 4-(4-Aminomethyl-phenyl)-1-(4-fluor-phenyl)-3-[3-(4-fluor-phenyl)-3-hydroxy-propyl]-azetidin-2-on (17), 48 µl Diisopropylethylamin in 1 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1 % Trifluoressigsäure) = 80/20 -> 10/90) getrennt. Man erhält das Produkt mit einem Molekulargewicht von 544.58 (C₂₇H₂₆F₂N₂O₆S₁); MS (ESI) 527.10 (M + H⁺ -H₂O)

### Beispiel IX

### {4-[3-[3-(4-Fluor-phenyl)-3-hydroxy-propyl]-2-(4-methoxy-phenyl)-4-oxo-azetidin-1-yl]-benzylcarbamoyl}-methanesulfonsäure (19):

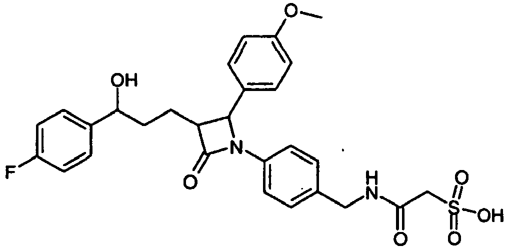

Zu einer Lösung aus 20 mg Sulfoessigsäure, 55 µl Diisopropylcarbodiimid, 38 mg Hydroxybenzotriazol in 1 ml Dimethylformamid wird eine Lösung aus 60 mg 1-(4-Aminomethyl-phenyl)-3-[3-(4-fluor-phenyl)-3-hydroxy-propyl]-4-(4-methoxy-phenyl)-azetidin-2-on (5) in 1 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1% Trifluoressigsäure) = 80/20 -> 10/90) getrennt. Man erhält das Produkt mit einem Molekulargewicht von 556.61 (C₂₈H₂₉F₁N₂O₇S₁); MS (ESI) 539.05 (M + H⁺ - H₂O)

### Beispiel X

### N-(4-{1-(4-Fluor-phenyl)-3-[3-(4-fluor-phenyl)-3-hydroxy-propyl]-4-oxo-azetidin-2-yl}-benzyl)-succinaminsäure (20):

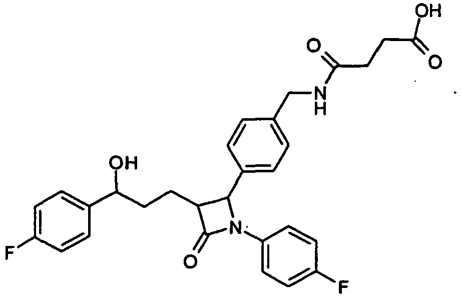

Zu einer Lösung aus 279 mg Bernsteinsäure, 92 µl Diisopropylcarbodiimid, 80 mg Hydroxybenzotriazol in 2 ml Dimethylformamid wird eine Lösung aus 100 mg 4-(4-Aminomethyl-phenyl)-1-(4-fluor-phenyl)-3-[3-(4-fluor-phenyl)-3-hydroxy-propyl]-azetidin-2-on (17), 33 µl Triethylamin in 2 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1% Trifluoressigsäure) = 80/20 -> 10/90) getrennt. Man erhält das Produkt mit einem Molekulargewicht von 522.55 (C₂₇H₂₆F₂N₂O₆S₁); MS (ESI) 545.19 (M + Na⁺)

### Beispiel XI

### {2-[2-({4-[3-[3-(4-Fluor-phenyl)-3-hydroxy-propyl]-2-(4-methoxy-phenyl)-4-oxo-azetidin-1- yl]-benzylcarbamoyl}-methoxy)-ethoxy]-ethoxy}-essigsäure (21):

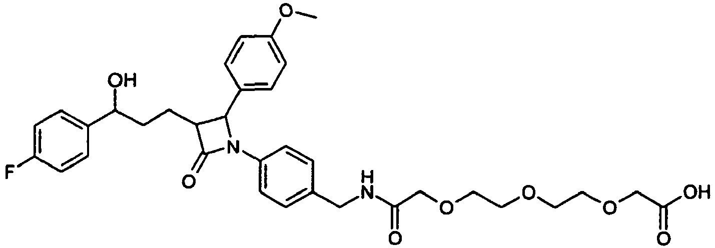

Zu einer Lösung aus 327 mg 3,6,9-Trioxaundecandisäure, 57 µl Diisopropylcarbodiimid, 50 mg Hydroxybenzotriazol in 2 ml Dimethylformamid wird eine Lösung aus 64 mg 1-(4-Aminomethyl-phenyl)-3-[3-(4-fluor-phenyl)-3-hydroxypropyl]-4-(4-methoxy-phenyl)- azetidin-2-on (5) , 21 µl Triethylamin in 1 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1 % Trifluoressigsäure) = 80/20 -> 10/90) getrennt. Man erhält das Produkt mit einem Molekulargewicht von 638,70 (C₃₄H₃₉F₁N₂O₉); MS (ESI) 639.27 (M + H⁺)

### Beispiel XII

### 4-((3-Carboxy-propionyl)-{4-[3-[3-(4-fluor-phenyl)-3-hydroxy-propyl]-2-(4-methoxyphenyl}4-oxo-azetidin-1-yl]-benzyl}-amino)-4-oxo-buttersäure (22):

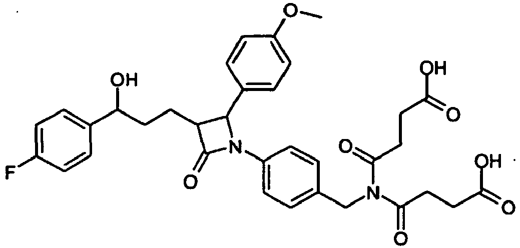

Zu einer Lösung aus 190 mg Bernsteinsäure, 63 µl Diisopropylcarbodiimid, 55 mg Hydroxybenzotriazol in 2 ml Dimethylformamid wird eine Lösung aus 70 mg 1-(4-Aminomethyl-phenyl)-3-[3-(4-fluor-phenyl)-3-hydroxy-propyl]-4-(4-methoxy-phenyl)-azetidin-2-on (5) , 23 µl Triethylamin in 1 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1% Trifluoressigsäure) = 80/20 -> 10/90) getrennt. Man erhält das Produkt mit einem Molekulargewicht von 634.4 (C₃₄H₃₅F₁N₂O₉); MS (ESI-neg.) 633.22 (M - H⁺)

### Beispiel XIII

### 11-{4-[3-[3-(4-Fluor-phenyl)-3-hydroxy-propyl]-2-(4-Methoxy-phenyl)-4-oxo-azetidin-1-yl]-benzylcarbamoyl}-undecansäure (23):

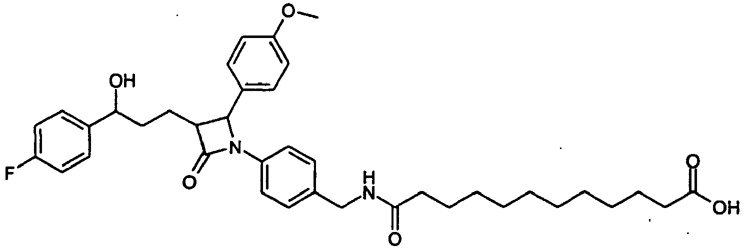

Zu einer Lösung aus 371 mg Dodecandisäure, 63 µl Diisopropylcarbodiimid, 55 mg Hydroxybenzotriazol in 2 ml Dimethylformamid wird eine Lösung aus 70 mg 1-(4-Aminomethyl-phenyl)-3-[3-(4-fluor-phenyl)-3-hydroxy-propyl]-4-(4-methoxy-phenyl)-azetidin-2-on (5) , 23 µl Triethylamin in 1 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1% Trifluoressigsäure) = 80/20 -> 10/90) getrennt. Man erhält das Produkt mit einem Molekulargewicht von 646.81 (C₃₈H₄₇F₁N₂O₆); MS (ESI) 647.35 (M + H⁺)

**Tabelle 1: Verbindungen der Formel I**

| | | | | | |
|---|---|---|---|---|---|
| 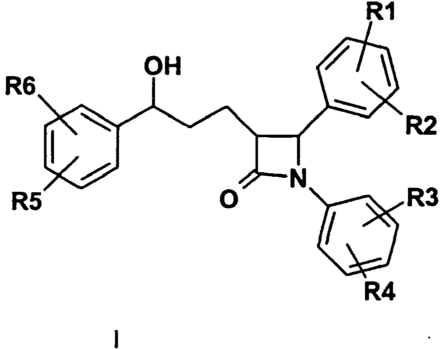 | | | | | |

| **Bsp.** | **R1, R2** | **R3, R4** | **R5, R6** | **Molekula r-gewicht der freien Base bzw. Säure (berechn et)** | **Molekul argewicht (gefund en)** |
|---|---|---|---|---|---|
| XIV | 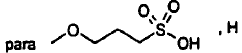 | para-F, H | para-F, H | 531,58 | 532,4 (MH⁺) |
| XV | 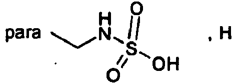 | para-F, H | para-F, H | 502,54 | 503,3 (MH⁺) |
| XVI | para-F, H | 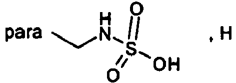 | para-F, H | 514,58 | 515,4 (MH⁺) |
| XVII | para-O-CH₃, H | 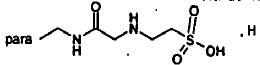 | para-F, H | 599,68 | 599,21 (M⁺) |
| XVIII | para-O-CH₃, H | 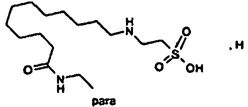 | para-F, H | 739,95 | 740,42 (MH⁺) |
| XIX | para-O-CH₃, H | 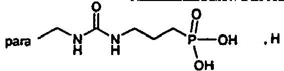 | para-F, H | 599,60 | 600,34 (MH⁺) |
| XX | para-O-CH₃, H | 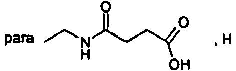 | para-F, H | 534,59 | 534,4 (MH⁺) |
| XXI | 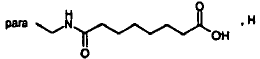 | para-F, H | para-F, H | 578,66 | 561,25 (MH⁺-H₂O) |
| XXII | 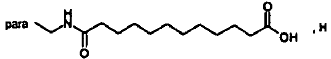 | para-F, H | para-F, H | 634,77 | 617,31 (MH⁺-H₂O) |
| XXIII | para-F, H | 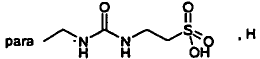 | para-F, H | 585,65 | 567,70 (MH⁺-H₂O) |
| XXIV | para-O-CH₃, H | 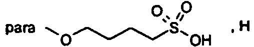 | para-F, H | 557.64 | 557.19 (M⁺) |
| XXV | 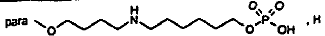 | Para-F, H | para-F, H | 660.70 | 660.28 (M⁺) |
| XXVI | para-O-CH₃, H | 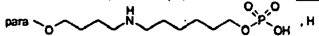 | para-F, H | 600.62 | 600.24 (M⁺) |
| XXVII | para-O-CH₃, H | 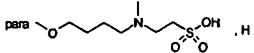 | para-F, H | 614.73 | . 597.32 (M-H₂O)⁺¹ |
| XXVIII | 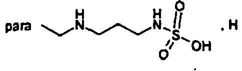 | para-F, H | para-F, H | 559,64 | 560,4 (MH⁺) |
| XXIX | 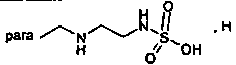 | para-F, H | para-F, H | 545,61 | 546,3 (MH⁺) |
| XXX | 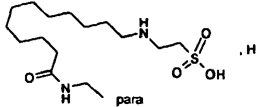 | para-F, H | para-F, H | 727,91 | 710,23 (MH⁺-H₂O) |
| XXXI | para-O-CH₃, H | 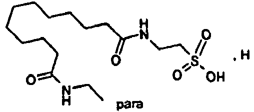 | para-F, H | 753,93 | 752,32 (M-H⁺); gemess en im Negativ-modus) |
| XXXII | 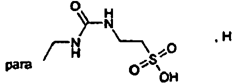 | para-F, H | para-F, H | 573,62 | 572,09 (M-H+); gemess en im Negativ-modus) |
| XXXIII | 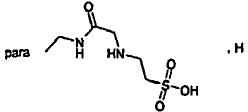 | para-F, H | para-F, H | 587,67 | 586,18 (M-H+); gemess en im Negativ-moduls) |

Die erfindungsgemäßen Verbindungen der Formel I wurden mit der nachfolgend beschriebenen Methode auf ihre Wirkung geprüft:
**Beeinflussung der Cholesterolabsorption + ³H- Taurocholsäureausscheidung anhand der fäkalen Ausscheidung an der Maus, Ratte oder Hamster**

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid ® 20 (Pharmacia- Upjohn) ((Spikung mit 0,25 µCi ¹⁴C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen) oder geeignetem Vehikel angesetzt.
Das Applikationsvolumen der Testsubstanz beträgt 0,5 ml/Maus. Die Testsubstanz wird unmittelbar vor der Testmahlzeit (Intralipid mit ¹⁴C-Cholesterol-label) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C-Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H20 und ¹⁴C- CO2 wird hochgerechnet auf die ausgeschiedene Menge an ³H-Taurocholsäure bzw. ¹⁴C-Cholesterol (Dual-Isotopen-Technik ). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

Die folgenden ED₅₀-Werte belegen die Aktivität der erfindungsgemäßen Verbindungen der Formel I

| Beispiel Nr. | ED₅₀ (Leber) [mg/Maus] |
|---|---|
| I | 1.0 |
| II | > 0.1 |
| IV | 0.3 |
| VIII | 0.3 |
| IX | < 1.0 |
| X | < 1.0 |
| XIII | < 0.1 |
| XVIII | 0.005 |
| XXI | 0.1 |
| XXII | 0.1 |
| XXV | 0.3 |
| XXVIII | 0.3 |

Aus der Tabelle ist abzulesen, daß die Verbindungen der Formel I eine sehr gute Cholesterin senkende Wirkung besitzen.

### Resorbierbarkeit:

Die Resorbierbarkeit der Verbindungen der Formel I wurde Caco-Zellmodell geprüft (A.R. Hilgers et al., Caco-2 cell monolayers as a model for drug transport across the intestinal mucosa, Pharm. Res. 1990 , 7, 902).

Aus den Meßdaten ist abzulesen, daß die erfindungsgemäßen Verbindungen der Formel I gegenüber den im Stand der Technik beschriebenen Verbindungen (Referenzstruktur) eine deutlich geringere Resorption aufweisen:

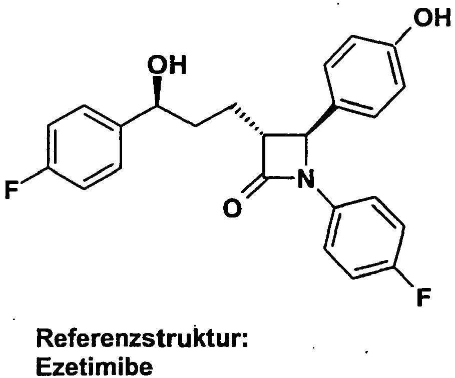

## Patentansprüche

1. Verbindungen der Formel I, 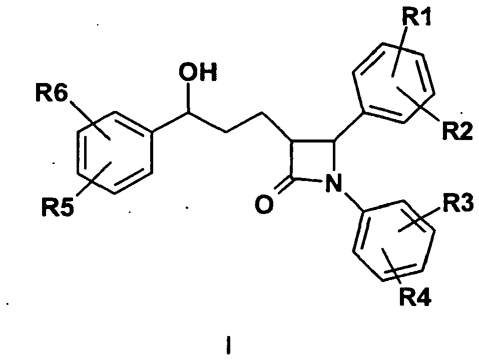 worin bedeuten
R1, R2, R3, R4, R5, R6 unabhängig voneinander (C₀-C₃₀)-Alkylen-(LAG)ₙ, wobei n = 1 - 5 sein kann und wobei ein oder mehrere C-Atome des Alkylenrests durch -S(O)ₙ-, mit n = 0 - 2, -O-, -(C=O)-, -(C=S)-, -CH=CH-, -CΞC-, -N((C₁-C₆-Alkyl)-, -N(Phenyl)-, -N((C₁-C₆)-Alkyl-Phenyl)-, -N(CO-(CH₂)₁-₁₀-COOH)- oder -NH- ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkiny), O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
(LAG)ₙ -(CH₂)₁₋₁₀-SO₃H, -(CH₂)₀₋₁₀-P(O)(OH)₂, (CH₂)₀₋₁₀-O-P(O)(OH)₂, -(CH₂)₀₋₁₀-COOH und n = 1 - 5 sein kann;
wobei immer mindestens einer der Reste R1 bis R6 die Bedeutung (C₀-C₃₀)-Alkylen-(LAG)ₙ, wobei n = 1 - 5 ist und wobei ein oder mehrere C-Atome des Alkylenrests durch -S(O)ₙ-, mit n = 0 - 2, -O-, -(C=O)-, -(C=S)-, -CH=CH-, -CΞC-, - N((C₁-C₆)-Alkyl)-, -N(Phenyl)-, -N((C₁-C₆)-Alkyl-Phenyl)-, -N(CO-(CH₂)₁₋₁₀-COOH)- oder -NH- ersetzt sein können,
besitzen muß,
sowie deren pharmazeutisch verträglichen Salze;
wobei die Verbindung 2-{[4-(4-{1-(4-Fluor-phenyl)-3-[3-(4-fluor-phenyl)-3-hydroxypropyl]-4-oxo-azetidin-2-yl}-phenoxy)-butyl]-methyl-amino}-ethansulfonsäure sowie solche Verbindungen, bei welchen die Reste R1 - R6 die Bedeutung -O-(CH₂)₁₋₁₀-COOH, (C₁-C₆)-Alkylen-COOH oder-COOH haben, ausgenommen sind.

2. Verbindungen der Formel I, gemäß Anspruch 1, **dadurch gekennzeichnet, daß** darin bedeuten
R2, R4, R5, R6 unabhängig voneinander H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁C₆)-Alkyl, S-(CH₂)ₙ-Phenyl, SO-(C₁-C₆)-Alkyl, SO-(CH₂)ₙ-Phenyl, SO-(C₁-C₆)-Alkyl, SO-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Pheny), wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1, R3 unabhängig voneinander (C₀-C₃₀)-Alkylen-(LAG) und wobei ein oder mehrere C-Atome des Alkylenrests durch -O-, -(C=O)-, -N(CH₃)- oder - NH- ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C6)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
(LAG) -(CH₂)₁₋₁₀-SO₃H, -(CH₂)₀₋₁₀-P(O)(OH)₂, (CH₂)₀₋₁₀-O-P(O)(OH)₂, -(CH₂)₀₋₁₀-COOH;
wobei immer mindestens einer der Reste R1 oder R3 die Bedeutung (C₀-C₃₀)-Alkylen-(LAG) und wobei ein oder mehrere C-Atome des Alkylenrests durch - O-, -(C=O)-, -N(CH₃)- oder -NH- ersetzt sein können;
besitzen muß,
sowie deren pharmazeutisch verträglichen Salze;
wobei die Verbindung 2-{[4-(4-{1-(4-Fluor-phenyl)-3-[3-(4-fluor-phenyl)-3-hydroxypropyl]-4-oxo-azetidin-2-yl}phenoxy)-butyl]-methyl-amino}-ethansulfonsäure sowie solche Verbindungen, bei welchen die Reste R1 - R6 die Bedeutung -O-(CH₂)₁₋₁₀-COOH, (C₁-C₆)-Alkylen-COOH oder -COOH haben, ausgenommen sind.

3. Verbindungen der Formel I, gemäß Anspruch 1 oder 2, **dadurch gekennzeichnet, daß** darin bedeuten
R2, R4, R5, R6 unabhängig voneinander H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alky), SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1, R3 unabhängig voneinander -(CH₂)₀₋₁-Y-W-(C₀-C₂₅)-Alkylen-Y'-W'-(LAG), worin ein oder mehrere C-Atome des Alkylenrests durch -O- ersetzt sein können
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann; wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
Y, W, Y' W' unabhängig voneinander NH, NCH₃, C=O, O, eine Bindung oder S(O)ₙ, mit n = 0 - 2;
oder Y-W oder Y'-W' jeweils zusammen genommen eine Bindung.
(LAG) -(CH₂)₁₋₁₀-SO₃H, -(CH₂)₀₋₁₀-P(O)(OH)₂, (CH₂)₀₋₁₀-O-P(O)(OH)₂, -(CH₂)₀₋₁₀-COOH;
wobei immer mindestens einer der Reste R1 oder R3 die Bedeutung -(CH₂)₀₋₁-Y-W-(C₀-C₂₅)-Alkylen-Y'-W'-(LAG), worin ein oder mehrere C-Atome des Alkylenrests durch -O- ersetzt sein können;
besitzen muß,
sowie deren pharmazeutisch verträglichen Salze;
wobei die Verbindung 2-{[4-(4-{1-(4-Fluor-phenyl)-3-[3-(4-fluor-phenyl)-3-hydroxypropyl]-4-oxo-azetidin-2-yl}-phenoxy)-butyl]-methyl-amino}-ethansulfonsäure sowie solche Verbindungen, bei welchen die Reste R1 - R6 die Bedeutung -O-(CH₂)₁₋₁₀-COOH, (C₁-C₆)-Alkylen-COOH oder -COOH haben, ausgenommen sind.

4. Verbindungen der Formel I gemäß einem oder mehreren der Ansprüche 1 bis 3, **dadurch gekennzeichnet, daß** darin bedeuten
(LAG) Carbonsäurerest oder ein Sulfonsäurerest;
sowie deren pharmazeutisch verträglichen Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und mindestens einen weiteren Wirkstoff.

7. Arzneimittel, gemäß Anspruch 6, **dadurch gekennzeichnet, daß** es als weiteren Wirkstoff eine oder mehrere Verbindungen, die den Lipidstoffwechsel normalisieren, enthält.

8. Arzneimittel, gemäß Anspruch 6 oder 7, **dadurch gekennzeichnet, daß** es als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylhamstoffe, Biguanide, Meglitinide, Thiazolidindione, *α*-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, *β*-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-*β-*Agonisten oder Amphetamine enthält.

9. Verbindungen gemäß einem oder mehreren der Ansprüche 1 bis 4 zur Anwendung als Medikament zur Behandlung von Lipidstoffwechselstörungen.

10. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen gemäß einem oder mehreren der Ansprüche 1 bis 4 zur Herstellung eines Medikaments zur Behandlung von Hyperlipidämie.

12. Verwendung der Verbindungen gemäß einem oder mehreren der Ansprüche 1 bis 4 zur Herstellung eines Medikaments zur Senkung des Serumcholesterinspiegels.

13. Verwendung der Verbindungen gemäß einem oder mehreren der Ansprüche 1 bis 4 zur Herstellung eines Medikaments zur Behandlung arteriosklerotischer Erscheinungen.

14. Verwendung der Verbindungen gemäß einem oder mehreren der Ansprüche 1 bis 4 zur Herstellung eines Medikaments zur Behandlung von Insulin Resistenz.

## Claims

1. A compound of the formula I, 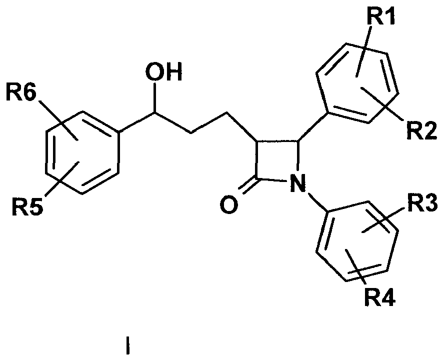 in which
R1, R2, R3, R4, R5, R6 independently of one another are (C₀-C₃₀)-alkylene-(LAG)ₙ, where n may be 1 - 5 and where one or more carbon atoms of the alkylene radical may be replaced by -S(O)ₙ-, where n = 0 - 2, -O-, -(C=O)-, -(C=S)-, -CH=CH-, -CΞC-, -N((C₁-C₆)-alkyl)-, -N(phenyl), -N((C₁-C₆)-alkyl-phenyl)- , -N(CO-(CH₂)₁₋₁₀-COOH)- or -NH-;
H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine; C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, O-(CH₂)ₙ-phenyl, where n may be 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
(LAG)ₙ is -(CH₂)₁₋₁₀-SO₃H, -(CH₂)₀₋₁₀-P(O)(OH)₂, -(CH₂)₀₋₁₀-O-P(O)(OH)₂, -(CH₂)₀₋₁₀-COOH and n may be 1 - 5;
where in each case at least one of the radicals R1 to R6 must have the meaning (C₀-C₃₀)-alkylene-(LAG)ₙ, where n = 1 - 5 and where one or more carbon atoms of the alkylene radical may be replaced by -S(O)ₙ-, where n = 0 - 2, -O-, -(C=O)-, -(C=S)-, -CH=CH-, -CΞC-, -N((C₁-C₆)-alkyl)-, -N(phenyl)-, -N((C₁-C₆)-alkyl-phenyl)- , -N(CO-(CH₂)₁₋₁₀-COOH)- or -NH-,
and its pharmaceutically acceptable salts;
except for the compound 2-{[4-(4-{1-(4-fluorophenyl)-3-[3-(4-fluorophenyl)-3-hydroxypropyl]-4-oxoazetidin-2-yl}phenoxy)butyl]methylamino}ethanesulfonic acid and those compounds in which the radicals R1 - R6 have the meaning -O-(CH₂)₁₋₁₀-COOH, (C₁-C₆)-alkylene-COOH or -COOH.

2. A compound of the formula I as claimed in claim 1, wherein
R2, R4, R5, R6 independently of one another are H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine; C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n may be 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, O-(CH₂)ₙ-phenyl, where n may be 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1, R3 independently of one another are (C₀-C₃₀)-alkylene-(LAG) and where one or more carbon atoms of the alkylene radical may be replaced by -O-, -(C=O)-, -N(CH₃)- or -NH-,
H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n may be 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n may be 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
(LAG) is -(CH₂)₁₋₁₀-SO₃H, -(CH₂)₀₋₁₀-P(O)(OH)₂, -(CH₂)₀₋₁₀-O-P(O)(OH)2, -(CH₂)₀₋₁₀-COOH;
where in each case at least one of the radicals R1 or R3 must have the meaning(C₀-C₃₀)-alkylene-(LAG) and where one or more carbon atoms of the alkylene radicalmay be replaced by -O-, -(C=O)-, -N(CH₃)- or -NH-;and its pharmaceutically acceptable salts;
except for the compound 2-{[4-(4-{1-(4-fluorophenyl)-3-[3-(4-fluorophenyl)-3-hydroxypropyl]-4-oxoazetidin-2-yl}phenoxy)butyl]methylamino}ethanesulfonic acidand those compounds in which the radicals R1 - R6 have the meaning-O-(CH₂)₁₋₁₀-COOH, (C₁-C₆)-alkylene-COOH or -COOH.

3. A compound of the formula I as claimed in claim 1 or 2, wherein
R2, R4, R5, R6 independently of one another are H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, S0₂-(C₁-C₆)-alkyl, SO₂-(CH₂)ₙ-phenyl, where n may be 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n may be 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1, R3 independently of one another are -(CH₂)₀₋₁-Y-W-(C₀-C₂₅)-alkylene-Y'-W'-(LAG), where one or more carbon atoms of the alkylene radical may be replaced by -O-;
H, F, Cl, Br, I, CF₃, NO₂, N₃, CN, COOH, COO(C₁-C₆)-alkyl, CONH₂, CONH(C₁-C₆)-alkyl, CON[(C₁-C₆)-a)kyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n may be 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, O-(CH₂)ₙ-phenyl, where n may be 0 - 6, the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
Y, W, Y' W' independently of one another are NH, NCH₃, C=O, O, a bond or S(O)ₙ, where n = 0 - 2;
or Y-W or Y'-W' in each case together are a bond.
(LAG) is -(CH₂)₁₋₁₀-SO₃H, -(CH₂)₀₋₁₀-P(O)(OH)₂, (CH₂)₀₋₁₀-O-P(O)(OH)₂, -(CH₂)₀₋₁₀-COOH;
where in each case at least one of the radicals R1 or R3 must have the meaning -(CH₂)₀₋₁-Y-W-(C₀-C₂₅)-alkylene-Y'-W'-(LAG), where one or more carbon atoms of the alkylene radical may be replaced by -O-;
and its pharmaceutically acceptable salts;
except for the compound 2-{[4-(4-{1-(4-fluorophenyl)-3-[3-(4-fluorophenyl)-3-hydroxypropyl]-4-oxoazetidin-2-yl}phenoxy)butyl]methylamino}ethanesulfonic acid and those compounds in which the radicals R1 - R6 have the meaning -O-(CH2)₁₋₁₀-COOH, (C₁-C₆)-alkylene-COOH or -COOH.

4. A compound of the formula I as claimed in one or more of claims 1 to 3, wherein (LAG) is a carboxylic acid radical or a sulfonic acid radical,
and its pharmaceutically acceptable salts.

5. A medicament comprising one or more compounds as claimed in one or more of claims 1 to 4.

6. A medicament comprising one or more compounds as claimed in one or more of claims 1 to 4 and at least one further active compound.

7. The medicament as claimed in claim 6, comprising, as further active compound, one or more compounds which normalize lipid metabolism.

8. The medicament as claimed in claim 6 or 7, which comprises, as further active compound, one or more
antidiabetics, hypoglycemically active compounds, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active compounds which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin-reuptake inhibitors, mixed serotonin and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists (bromocriptine, doprexin), lipase/amylase inhibitors, PPAR modulators, RXR modulators or TR-β-agonists or amphetamines.

9. A compound as claimed in one or more of claims 1 to 4 for use as a medicament for the treatment of impaired lipid metabolism.

10. A process for preparing a medicament comprising one or more of the compounds as claimed in one or more of claims 1 to 4, which comprises mixing the active compound with a pharmaceutically acceptable carrier and bringing this mixture into a form suitable for administration.

11. The use of the compounds as claimed in one or more of claims 1 to 4 for preparing a medicament for treating hyperlipidemia.

12. The use of the compounds as claimed in one or more of claims 1 to 4 for preparing a medicament for lowering the serum cholesterol concentration.

13. The use of the compounds as claimed in one or more of claims 1 to 4 for preparing a medicament for treating arteriosclerotic manifestations.

14. The use of the compounds as claimed in one or more of claims 1 to 4 for preparing a medicament for treating insulin resistance.

## Revendications

1. Composés de formule I, 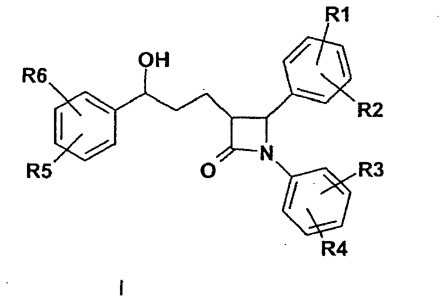 dans laquelle
R1, R2, R3, R4, R5, R6 sont indépendamment les uns des autres un alkylène-(LAG)ₙ en (C₀-C₃₀), où n peut être 1 à 5 et où un ou plusieurs atomes de carbone du radical alkylène peuvent être remplacés par -S(O)ₙ-, où n = 0 à 2, -O-, -(C=O)-, -(C=S)-, -CH=CH-, -C≡C-, -N(alkyle en (C₁-C₆))-, -N(phényle), -N (alkylphényle en (C₁-C₆))-, -N(CO-(CH₂)₁₋₁₀-COOH)- ou -NH-;
H, F, Cl, Br, I, CF₃, NO₂, N₃, CN, COOH, COO-alkyle en (C₁-C₆), CONH₂, CONH-alkyle en (C₁-C₆), CON[alkyle en (C₁-C₆)]₂, alkyle en (C₁-C₆), alcényle en (C₂-C₆), alcynyle en (C₂-C₆), O-alkyle en (C₁-C₆), où un, plusieurs ou tous les hydrogènes dans les radicaux alkyle peuvent être remplacés par du fluor ;
C(=NH)(NH₂), PO₃H₂, SO₃H, SO₂-NH₂, SO₂NH-alkyle en (C₁-C₆), SO₂N[alkyle en (C₁-C₆)]₂, S-alkyle en (C₁-C₆), S-(CH₂)ₙ-phényle, SO-alkyle en (C₁-C₆), SO-(CH₂)ₙ-phényle, SO₂-alkyle en (C₁-C₆), SO₂-(CH₂)ₙ-phényle, où n = 0 à 6 et le radical phényle peut être substitué jusqu'à deux fois par F, Cl, Br, OH, CF₃, NO₂, CN, OCF₃, O-alkyle en (C₁-C₆), alkyle en (C₁-C₆), NH₂ ;
NH₂, NH-alkyle en (C₁-C₆), N (alkyle en (C₁-C₆))₂, NH(acyle en (C₁-C₇)), phényle, O-(CH₂)ₙ-phényle, où n peut être 0 à 6, où le noyau phényle peut être mono- à trisubstitué par F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SO₂-CH₃, COOH, COO-alkyle en (C₁-C₆), CONH₂ ;
(LAG)ₙ est - (CH₂)₁₋₁₀-SO₃H, - (CH₂)₀₋₁₀-P(O) (OH)₂, (CH₂)₀₋₁₀-O-P(O) (OH)₂, - (CH₂)₀₋₁₀-COOH et n peut être 1 à 5 ;
dans laquelle dans chaque cas, au moins un des radicaux R1 à R6 doit avoir la signification alkylène-(LAG)ₙ en (C₀-C₃₀), où n = 1 à 5 et dans lequel un ou plusieurs atomes de carbone du radical alkylène peuvent être remplacés par -S(O)ₙ-, dans lequel n = 0 à 2, -O-, -(C=O)-, -(C=S)-, -CH=CH-, -C≡C-, -N(alkyle en (C₁-C₆))-, -N(phényle)-, -N(alkylphényle en (C₁-C₆))-, -N (CO- (CH₂)₁₋₁₀-COOH) - ou -NH-,
et leurs sels pharmaceutiquement acceptables ;
excepté pour le composé acide 2-{[4-(4-{1-(4-fluorophényl)-3-[3-(4-fluorophényl)-3-hydroxypropyl]-4-oxoazétidin-2-yl}phénoxy)butyl]méthylamino}éthanesulfonique et les composés dans lesquels les radicaux R1 à R6 ont la signification -O-(CH₂)₁₋₁₀-COOH, alkylène en (C₁-C₆)-COOH ou -COOH.

2. Composés de formule I selon la revendication 1, **caractérisés en ce que**
R2, R4, R5, R6 sont indépendamment les uns des autres H, F, Cl, Br, I, CF₃, NO₂, N₃, CN, COOH, COO-alkyle en (C₁-C₆), CONH₂, CONH-alkyle en (C₁-C₆), CON[alkyle en (C₁-C₆)]₂, alkyle en (C₁-C₆), alcényle en (C₂-C₆), alcynyle en (C₂-C₆), O-alkyle en (C₁-C₆), où un, plusieurs ou tous les hydrogènes dans les radicaux alkyle peuvent être remplacés par du fluor ; C(=NH)(NH₂), PO₃H₂, SO₃H, SO₂-NH2, SO₂NH-alkyle en (C₁-C₆), SO₂N[alkyle en (C₁-C₆)]₂, S-alkyle en (C₁-C₆), S-(CH₂)ₙ-phényle, SO-alkyle en (C₁-C₆), SO-(CH₂)ₙ-phényle, SO₂-alkyle en (C₁-C₆), SO₂-(CH₂)ₙ-phényle, où n peut être 0 à 6 et le radical phényle peut être substitué jusqu'à deux fois par F, Cl, Br, OH, CF₃, NO₂, CN, OCF₃, 0-alkyle en (C₁-C₆), alkyle en (C₁-C₆), NH₂ ;
NH₂, NH-alkyle en (C₁-C₆), N (alkyle en (C₁-C₆))₂, NH(acyle en (C₁-C₇)), phényle, O-(CH₂)ₙ-phényle, où n peut être 0 à 6, où le noyau phényle peut être mono- à trisubstitué par F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SO₂-CH₃, COOH, COO-alkyle en (C₁-C₆), CONH₂ ;
R1 , R3 sont indépendamment l'un de l'autre un alkylène-(LAG) en (C₀-C₃₀) et où un ou plusieurs atomes de carbone du radical alkylène peuvent être remplacés par -O-, -(C=O)-, -N(CH₃)- ou -NH-,
H, F, Cl, Br, I, CF₃, NO₂, N₃, CN, COOH, COO-alkyle en (C₁-C₆), CONH₂, CONH-alkyle en (C₁-C₆), CON[alkyle en (C₁-C₆)]₂, alkyle en (C₁-C₆), alcényle en (C₂-C₆), alcynyle en (C₂-C₆), O-alkyle en (C₁-C₆), où un, plusieurs ou tous les hydrogènes dans les radicaux alkyle peuvent être remplacés par du fluor ;
C(=NH)(NH₂), PO₃H₂, SO₃H, SO₂-NH₂, SO₂NH-alkyle en (C₁-C₆), SO₂N[alkyle en (C₁-C₆)]₂, S-alkyle en (C₁-C₆), S-(CH₂)ₙ-phényle, SO-alkyle en (C₁-C₆), SO-(CH₂)ₙ-phényle, SO₂-alkyle en (C₁-C₆), SO₂-(CH₂)ₙ-phényle, où n peut être 0 à 6 et le radical phényle peut être substitué jusqu'à deux fois par F, Cl, Br, OH, CF₃, NO₂, CN, OCF₃, 0-alkyle en (C₁-C₆), alkyle en (C₁-C₆), NH₂ ;
NH₂, NH-alkyle en (C₁-C₆) , N (alkyle en (C₁-C₆))₂, NH (acyle en (C₁-C₇)), phényle, 0- (CH₂)ₙ-phényle, où n peut être 0 à 6, où le noyau phényle peut être mono- à trisubstitué par F, Cl, Br, I, OH, CF₃, NO₂, CN, OCF₃, O-alkyle en (C₁-C₆), alkyle en (C₁-C₆), NH₂, NH-alkyle en (C₁-C₆), N(alkyle en (C₁-C₆))₂, SO₂-CH₃, COOH, COO-alkyle en (C₁-C₆), CONH₂ ;
(LAG) est -(CH₂)₁₋₁₀-SO₃H, -(CH₂)₀₋₁₀-P(O)(OH)₂, (CH₂)₀₋₁₀-O-P(O)(OH)₂, -(CH₂)₀₋₁₀-COOH ;
dans laquelle dans chaque cas, au moins un des radicaux R1 ou R3 doit avoir la signification alkylène-(LAG) en (C₀-C₃₀) et où un ou plusieurs atomes de carbone du radical alkylène peuvent être remplacés par -O-, -(C=O)-, -N(CH₃)- ou -NH-;
et leurs sels pharmaceutiquement acceptables ;
excepté pour le composé acide 2-{[4-(4-{1-(4-fluorophényl)-3-[3-(4-fluorophényl)-3-hydroxypropyl]-4-oxoazétidin-2-yl}phénoxy)butyl]méthylamino}éthanesulfonique et les composés dans lesquels les radicaux R1 à R6 ont la signification -O- (CH₂)₁₋₁₀-COOH, alkylène en (C₁-C₆)-COOH ou -COOH.

3. Composés de formule I selon la revendication 1 ou 2, **caractérisés en ce que**
R2, R4, R5, R6 sont indépendamment les uns des autres H, F, Cl, Br, I, CF₃, NO₂, N₃, CN, COOH, COO-alkyle en (C₁-C₆), CONH₂, CONH-alkyle en (C₁-C₆), CON[alkyle en (C₁-C₆)]₂, alkyle en (C₁-C₆), alcényle en (C₂-C₆), alcynyle en (C₂-C₆), O-alkyle en (C₁-C₆), où un, plusieurs ou tous les hydrogènes dans les radicaux alkyle peuvent être remplacés par du fluor ;
C(=NH)(NH₂), PO₃H₂, SO₃H, SO₂-NH₂, SO₂NH-alkyle en (C₁-C₆), SO₂N[alkyle en (C₁-C₆)]₂, S-alkyle en (C₁-C₆), S-(CH₂)ₙ-phényle, SO-alkyle en (C₁-C₆), SO-(CH₂)ₙ-phényle, SO₂-alkyle en (C₁-C₆), SO₂-(CH₂)ₙ-phényle, où n peut être 0 à 6 et le radical phényle peut être substitué jusqu'à deux fois par F, Cl, Br, OH, CF₃, NO₂, CN, OCF₃, 0-alkyle en (C₁-C₆), alkyle en (C₁-C₆), NH₂ ;
NH₂, NH-alkyle en (C₁-C₆), N (alkyle en (C₁-C₆))₂, NH (acyle en (C₁-C₇), phényle, 0- (CH₂)ₙ-phényle, où n peut être 0 à 6, où le noyau phényle peut être mono- à trisubstitué par F, Cl, Br, I, OH, CF₃, NO₂, CN, OCF₃, O-alkyle en (C₁-C₆), alkyle en (C₁-C₆), NH₂, NH-alkyle en (C₁-C₆), N(alkyle en (C₁-C₆))₂, SO₂-CH₃, COOH, COO-alkyle en (C₁-C₆), CONH₂ ;
R1 , R3 sont indépendamment l'un de l'autre un -(CH2)₀₋₁-Y-W-alkylène-Y'-W'-(LAG) en (C₀-C₂₅) , où un ou plusieurs atomes de carbone du radical alkylène peuvent être remplacés par -O- ;
H, F, Cl, Br, I, CF₃, NO₂, N₃, CN, COOH, COO-alkyle en (C₁-C₆), CONH₂, CONH-alkyle en (C₁-C₆), CON[alkyle en (C₁-C₆)]₂, alkyle en (C₁-C₆), alcényle en (C₂-C₆), alcynyle en (C₂-C₆), O-alkyle en (C₁-C₆), où un, plusieurs ou tous les hydrogènes dans les radicaux alkyle peuvent être remplacés par du fluor ;
C(=NH)(NH₂), PO₃H2, SO₃H, SO₂-NH₂, SO₂NH-alkyle en (C₁-C₆), SO₂N[alkyle en (C₁-C₆)]₂, S-alkyle en (C₁-C₆), S-(CH₂)ₙ-phényle, SO-alkyle en (C₁-C₆), SO-(CH₂)ₙ-phényle, SO₂-alkyle en (C₁-C₆), SO₂- (CH₂)ₙ-phényle, où n peut être 0 à 6 et le radical phényle peut être substitué jusqu'à deux fois par F, Cl, Br, OH, CF₃, NO₂, CN, OCF₃, 0-alkyle en (C₁-C₆), alkyle en (C₁-C₆), NH₂ ;
NH₂, NH-alkyle en (C₁-C₆), N (alkyle en (C₁-C₆))₂, NH(acyle en (C₁-C₇)), phényle, O-(CH₂)ₙ-phényle, où n peut être 0 à 6, le noyau phényle peut être mono- à trisubstitué par F, Cl, Br, I, OH, CF₃, NO₂, CN, OCF₃, O-alkyle en (C₁-C₆), alkyle en (C₁-C₆), NH₂, NH-alkyle en (C₁-C₆), N(alkyle en (C₁-C₆))₂, SO₂-CH₃, COOH, COO-alkyle en (C₁-C₆), CONH₂ ;
Y, W, Y' et W' sont indépendamment l'un de l'autre NH, NCH₃, C=O, O, une liaison ou S(O)ₙ, où n = 0 à 2;
ou Y-W ou Y'-W' forment ensemble dans chaque cas une liaison.
(LAG) est -(CH₂)₁-₁₀-SO₃H, -(CH₂)₀₋₁₀-P(O)(OH)₂, (CH₂)₀₋₁₀-O-P(O)(OH)₂, -(CH₂)₀₋₁₀-COOH ;
dans laquelle, dans chaque cas, au moins un des radicaux R1 ou R3 doit avoir la signification - (CH₂)₀₋₁-Y-W-alkylène-Y'-W'-(LAG) en (C₀-C₂₅ ), et où un ou plusieurs atomes de carbone du radical alkylène peuvent être remplacés par -O- ;
et leurs sels pharmaceutiquement acceptables ;
excepté pour le composé acide 2-{[4-(4-{1-(4-fluorophényl)-3-[3-(4-fluorophényl)-3-hydroxypropyl]-4-oxoazétidin-2-yl}phénoxy)butyl]méthylamino}éthanesulfonique et les composés dans lesquels les radicaux R1 à R6 ont la signification -O-(CH₂)₁₋₁₀-COOH, alkylène (en C₁-C₆)-COOH ou -COOH.

4. Composés de formule I selon une ou plusieurs des revendications 1 à 3, **caractérisés en ce que** (LAG) est un radical d'acide carboxylique ou un radical d'acide sulfonique, et ses sels pharmaceutiquement acceptables.

5. Médicament comprenant un ou plusieurs composés selon une ou plusieurs des revendications 1 à 4.

6. Médicament comprenant un ou plusieurs composés selon une ou plusieurs des revendications 1 à 4 et au moins un autre composé actif.

7. Médicament selon la revendication 6, **caractérisé en ce qu'**il comprend, comme autre composé actif, un ou plusieurs composés qui normalisent le métabolisme lipidique.

8. Médicament selon la revendication 6 ou 7, **caractérisé en ce qu'**il comprend comme autre composé actif, un ou plusieurs antidiabétiques, composés hypoglycémiquement actifs, inhibiteurs de la HMGCoA réductase, inhibiteurs d'absorption du cholestérol, agonistes PPAR gamma, agonistes PPAR alpha, agonistes PPAR alpha/gamma, fibrates, inhibiteurs MTP, inhibiteurs d'absorption d'acide biliaire, inhibiteurs de CETP, absorbants d'acide biliaire polymères, inducteurs des récepteurs de LDL, inhibiteurs ACAT, antioxydants, inhibiteurs de la lipoprotéine lipase, inhibiteurs de l'ATP-citrate lyase, inhibiteurs de la squalène synthétase, antagonistes de la lipoprotéine (a), inhibiteurs de la lipase, insulines, sulfonylurées, biguanides, méglitinides, diones de thiazolidine, inhibiteurs de l'α-glucosidase, composés actifs qui agissent sur les canaux potassiques ATP-dépendants des cellules bêta, agonistes de CART, agonistes de NPY, agonistes des MC4, agonistes des orexines, agonistes de H3, agonistes de TNF, agonistes de CRF, antagonistes de CRF BP, agonistes d'urocortine, agonistes de β3, agonistes de MSH (hormone stimulant les mélanocytes), agonistes de CCK, inhibiteurs de la réabsorption de la sérotonine, composés mixtes sérotoninergiques et noradrénergiques, agonistes de 5HT, agonistes de la bombésine, antagonistes de la galanine, l'hormone de croissance, composés libérant l'hormone de croissance, agonistes de THR, modulateurs 2 ou 3 de la protéine découplante, agonistes de la leptine, agonistes de DA (bromocriptine, doprexine), inhibiteurs de la lipase/amylase, modulateurs des PPAR, modulateurs des récepteurs RXR ou des agonistes de TR-β ou amphétamines.

9. Composés selon une ou plusieurs des revendications 1 à 4 pour une utilisation en tant que médicament destiné à traiter les dysfonctionnements du métabolisme lipidique.

10. Procédé pour la préparation d'un médicament comprenant un ou plusieurs des composés selon une ou plusieurs des revendications 1 à 4, **caractérisé en ce qu'**il comprend le mélange du composé actif avec un excipient pharmaceutiquement acceptable et la mise en forme de ce mélange sous une forme appropriée pour l'administration.

11. Utilisation des composés selon une ou plusieurs des revendications 1 à 4 pour la préparation d'un médicament destiné à traiter l'hyperlipidémie.

12. Utilisation des composés selon une ou plusieurs des revendications 1 à 4 pour la préparation d'un médicament destiné à réduire la concentration de cholestérol sérique.

13. Utilisation des composés selon une ou plusieurs des revendications 1 à 4 pour la préparation d'un médicament destiné à traiter les manifestations artériosclérotiques.

14. Utilisation des composés selon une ou plusieurs des revendications 1 à 4 pour la préparation d'un médicament destiné à traiter la résistance à l'insuline.
